Europäisches Patentamt

(19) European Patent Office

Office européen des brevets

(11) Publication number: **0 120 840**
**B1**

(12) **EUROPEAN PATENT SPECIFICATION**

(45) Date of publication of patent specification: **22.03.89**

(51) Int. Cl.⁴: **A 61 F 13/00**

(21) Application number: **82903221.8**

(22) Date of filing: **28.09.82**

(86) International application number:
**PCT/US82/01347**

(87) International publication number:
**WO 84/01285 12.04.84 Gazette 84/10**

(54) SYSTEM AND METHOD FOR BANDAGING A PATIENT.

(43) Date of publication of application:
**10.10.84 Bulletin 84/41**

(45) Publication of the grant of the patent:
**22.03.89 Bulletin 89/12**

(84) Designated Contracting States:
**AT BE CH DE FR GB LI LU NL SE**

(56) References cited:
**EP-A-0 051 935**
**EP-A-0 066 899**
**US-A-3 236 370**
**US-A-3 349 765**
**US-A-4 080 963**
**US-A-4 374 520**

(73) Proprietor: **BAXTER INTERNATIONAL INC. (a Delaware corporation)**
**One Baxter Parkway**
**Deerfield Illinois 60015 (US)**

(72) Inventor: **GROSSMANN, Frederic**
**920 Larchmont Lane**
**Lake Forest, IL 60045 (US)**
Inventor: **SIMS, Larry A.**
**435 Second Street**
**Hermosa Beach, CA 90254 (US)**

(74) Representative: **Seaborn, George Stephen et al**
**c/o Edward Evans & Co. Chancery House 53-64**
**Chancery Lane**
**London WC2A 1SD (GB)**

Courier Press, Leamington Spa, England.

## Description

### Background

There has been a substantial problem in applying large area adhesive backed bandages to patients. Such bandages might be used for a surgical drape, such as shown in United States Patent 3,236,370. There is considerable difficulty in keeping the large area drapes from sticking to themselves or from wrinkling during the application procedure. Often several nurses or physicians are required to apply such surgical incise drapes. The drape is called an "incise" drape when a surgical incision is made directly through the drape. Similar problems exist with other relatively large area bandages for wounds or burns.

One type of adhesive backed bandage having a relatively large area making it difficult to handle is a vapour permeable, bacteria impermeable transparent bandage manufactured by the British firm of Smith & Nephew, Ltd. and marketed in the United States under the OP—SITE trademark. This OP—SITE bandage has had considerable problems because of its extremely flexible nature and tacky adhesive that tends to stick to itself and wrinkle if not very carefully handled when applied to a patient.

EP—A—0 066 899 (intermediate document according to art. 54(3) EPC) discloses a delivery system for adhesively affixed copolymer medical coverings such as adhesive bandages, surgical drapes and medical dressings, comprising the medical covering in the form of a film sheet disposed on and supported by a film sheet carrier, transfer adhesive being disposed on a release or transfer sheet which is itself positioned on the surface of the film sheet opposite the film sheet carrier, the adhesive being disposed between the film sheet and the release or transfer film, the adhesive remaining on the film sheet when the release or transfer sheet is removed therefrom prior to the film sheet being applied to a patient, and a tab being affixed to the back of the film sheet carrier to facilitate separation of the film sheet carrier from the film sheet after application of the film sheet to a patient.

EP—A—0 051 935 discloses a conformable polymeric film suitable for use as a surgical drape or covered dressing and having on one surface a coating of pressure-sensitive adhesive for use in attaching the film to a patient, a release liner covering the adhesive, and a releasable layer being attached to the other surface of the film in a more tenacious manner than the release liner is attached to the film.

### Summary of the invention

The present invention overcomes the above problems by utilizing a separate applicator handle that is joined to the bandage while such bandage is being applied to the patient, and this applicator handle is removed after the bandage has been applied so the bandage can be smoothly contoured to the patient's anatomy. Such applicator handle can be mechanically or adhesively secured to the bandage.

Accordingly the present invention provides a system for bandaging a patient comprising: a flexible bandage having a backing with an adhesive thereon; and a separate applicator handle that is substantially less flexible than the bandage and is joinable to such bandage for control of the bandage during application, said applicator handle being removable from the bandage so as not to interfere with the flexible functioning of the bandage on a patient's anatomy.

### The Drawings

Figure 1 is a top plan view of the flexible bandage;

Figure 2 is a top plan view of the flexible bandage with two applicator handles of a first embodiment attached;

Figure 3 is an enlarged sectional view taken along line 3—3 of Figure 1;

Figure 4 is an enlarged sectional view taken along line 4—4 of Figure 2;

Figure 5 is a left end view of Figure 4 showing a clamp type handle in closed condition;

Figure 6 is a view similar to Figure 5, but showing the clamp type handle in open condition;

Figure 7 is a schematic showing of a patient with an incise drape;

Figure 8 is a top plan view of a second embodiment of a handle attached to the flexible bandage;

Figure 9 is an enlarged sectional view taken along line 9—9 of Figure 8;

Figure 10 is a sectional view of the flexible drape applied to a patient with the applicator handles being removed;

Figure 11 is a perspective view of a third embodiment of the applicator handle showing a pair of the handles connected to the flexible bandage;

Figure 12 is a sectional view of the drape being applied to a patient with the applicator handles of Figure 11;

Figure 13 is a top plan view of a sterile package containing the flexible bandage and two applicator handles; and

Figure 14 is a top plan view of a sterile package containing an applicator handle.

### Detailed description

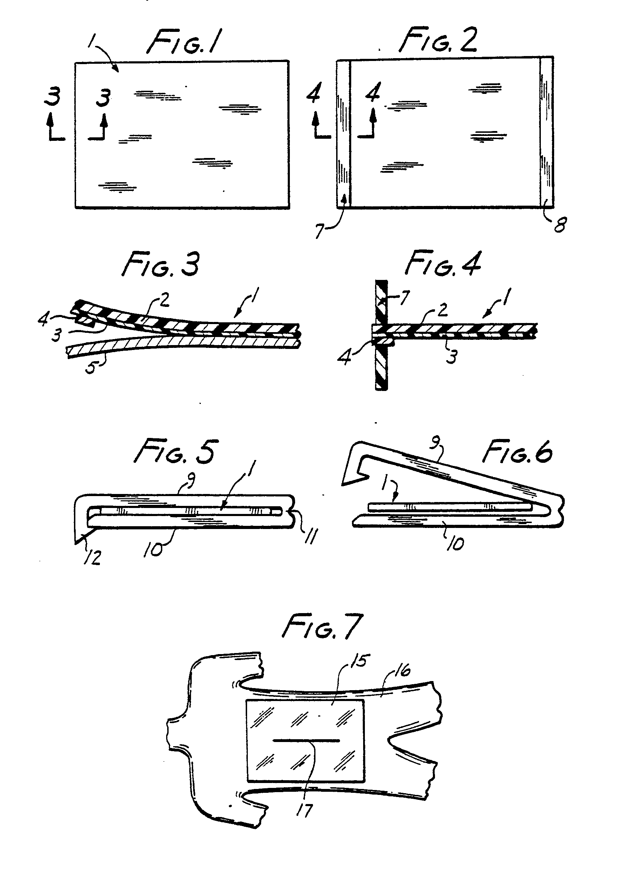

In Figures 1 and 3, a large area bandage, shown generally at 1, is illustrated. This bandage includes a backing 2 that has one surface coated with an adhesive 3. A nonadhesive strip 4 covers an outer edge portion of the adhesive 3 to prevent such outer edge portion from sticking to a removable liner 5, which protects the adhesive 3 until ready to use. Strip member 4 thus provides a tab section for grasping and pulling off the liner 5. Backing 2 and strip member 4 can be of a thermoplastic material, while removable liner 5 can be of a paper material. For use as a bandage, preferably the backing 2 and adhesive 3 are highly permeable to water vapour, but impermeable to liquid water and bacteria passage. Thus, a patient's covered wound, or body areas adjacent such wound, can breathe and receive oxygen through the bandage.

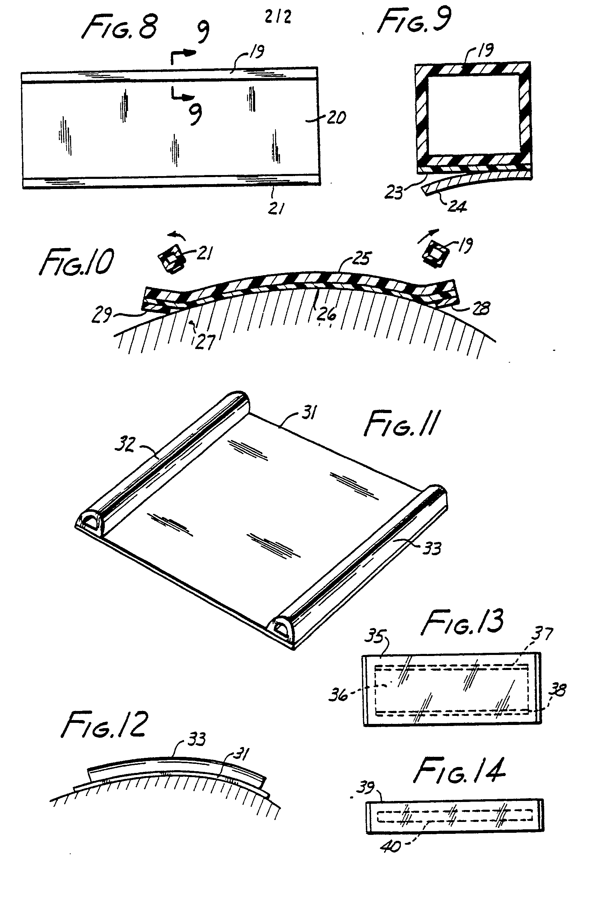

Figure 2 shows the flexible bandage with the protective liner 5 removed. As shown in Figures 2 and 4, a rigid handle, shown generally at 7, is clamped along a left edge of the flexible bandage of Figure 2. Also, a rigid handle 8 is clamped to the right edge portion of the flexible bandage in Figure 2. This rigid handle, shown in Figures 5 and 6, can include a pair of opposed jaws 9 and 10 that are connected by a hinge portion 11. Jaws 9 and 10 can be held in clamped position by a snap latch 12. Between jaws 9 and 10 in Figure 5 is clamped the flexible bandage, shown generally at 1.

It is acknowledged that hinged type rigid clamps with snap latches have been known and used for completely different purposes. For instance, such clamps have been used to hold upper edges of paper sheets for writing, clamp umbilical cords while they are being cut, etc. To applicants' knowledge, such a hinged clamp has never been used in a system which includes clamping an adhesive backed bandage during the application of such bandage to a patient and thereafter removal of such clamp, so as not to interfere with the flexible functioning of the bandage.

One example of the large area bandage is shown in Figure 7 where an incise drape 15 has been secured to a patient 16. A surgical incision 17 has been made through both the drape and the patient. The water vapor permeable and liquid and bacteria impermeable incise drape protects the patient from contamination adjacent the surgical wound.

In a second embodiment of the applicator handle shown in Figures 8 and 9, a rigid rectangular handle 19 is attached along one edge of a flexible bandage 20. A similar applicator handle 21 is attached to an opposite edge. It should be noted that in Figure 8 the applicator handles are along the longer edges of a rectangular bandage, whereas in Figure 2 the handles are along the shorter edges of a rectangular bandage. The applicator handles could be placed in either of these locations. In the enlarged sectional view of Figure 9, the rectangular tubular handle 19 has an adhesive coating 23 on one surface that is temporarily protected by a removable liner 24. In Figure 8, this liner 24 has been removed and the adhesive 23 has been secured to flexible drape 20.

When the adhesive backed flexible drape is applied to a patient, a patient's anatomy often has various curved configurations. As shown in Figure 10, the flexible bandage with a backing 25 and adhesive 26 has been attached to a patient, shown schematically in section at 27. Strip members 28 and 29 prevent the outermost edges of the bandage from adhering to the patient, thus making convenient tabs for grasping when removing the bandage from the patient. After the bandage has been applied to the patient, the rectangular tubular handles 19 and 21 are peeled from the back of the flexible bandage. This can be done by a rotating action.

Figure 11 shows still another embodiment in which the flexible bandage 31 is secured to opposed applicator handles 32 and 33, which are shown with flattened lower edges secured to bandage 31 by adhesive. The applicator handles of Figure 11 are formed of extruded tubing which can be somewhat flexible or bendable (although stiffer than the flexible bandage). Thus, as in Figure 12, when the flexible bandage is applied to a patient, handles 32 and 33 can temporarily conform to the patient's anatomy when sticking down the bandage. Handles 32 and 33 would be removed from such flexible bandage after the application step so as not to interfere with the flexible functioning of the bandage 31.

The flexible bandage and applicator handles can be packaged together in a sterile package such as 35 in Figure 13. Here flexible bandage 36 is packaged with applicator handles 37 and 38. These handles 37 and 38 can be either preattached to the flexible drape 36 or merely contained in the same package for connection to such bandage at the time of application to a patient. Alternatively, a sterile package 39 could contain one or more applicator handles, such as 40, for subsequent combining with a separate bandage.

Throughout the specification and claims the term "bandage" has been used in a broad sense to include various types of dressings and drapes which are adhesively applied to a patient.

**Claims**

1. A system for bandaging a patient (16, 27) comprising: a flexible bandage (1, 20, 31, 36) having a backing (2, 25) with an adhesive (3, 26) thereon; and a separate applicator handle (7, 8, 19, 21, 32, 33, 37, 38) that is substantially less flexible than the bandage and is joinable to such bandage for control of the bandage during application, said applicator handle being removable from the bandage so as not to interfere with the flexible functioning of the bandage on a patient's anatomy.

2. A system for bandaging a patient (16, 27) as set forth in Claim 1, wherein the flexible bandage (1, 20, 31, 36) is a dressing for a wound.

3. A system for bandaging a patient (16, 27) as set forth in Claim 2, wherein the flexible bandage (1, 20, 31, 36) is a burn dressing.

4. A system for bandaging a patient (16, 27) as set forth in Claim 1, wherein the flexible bandage (1, 20, 31, 36) is a surgical drape.

5. A system for bandaging a patient (16, 27) as set forth in Claim 4, wherein the flexible bandage (1, 20, 31, 36) is an incise surgical drape which is severable at a surgical incision (17).

6. A system for bandaging a patient (16, 27) as set forth in any preceding claim, wherein the bandage (1, 20, 31, 36) is permeable to water vapour, but impermeable to liquid water and bacteria.

7. A system for bandaging a patient (16, 27) as set forth in any preceding claim, wherein the backing (2, 25) and adhesive (3, 26) are transparent for viewing a patient (16) beneath the bandage.

8. A system for bandaging a patient (16, 27) as set forth in any preceding claim, wherein the adhesive (3, 26) forms a continuous coating on the backing (2, 25) so that the adhesive is adapted to secure the backing directly to a wound or incision area.

9. A system for bandaging a patient (16, 27) as set forth in any preceding claim, wherein the handle (7, 8, 19, 21) is rigid. -

10. A system for bandaging a patient (16) as set forth in any preceding claim, wherein the handle (7, 8) has a pair of movable jaws (9, 10) to temporarily grip the bandage (1).

11. A system for bandaging a patient (16) as set forth in Claim 10, wherein there is a latch means (12) on the jaws.

12. A system for bandaging a patient (27) as set forth in any of Claims 1 to 9, wherein the applicator handle (19, 21) is secured to the bandage (20) by an adhesive (23).

13. A system for bandaging a patient as set forth in any of claims 1 to 9 and 12, wherein the applicator handle (32, 33) is bendable.

14. A system for bandaging a patient (27) as set forth in any of Claims 1 to 9, 12 and 13, wherein the applicator handle (19, 21, 32, 33) is tubular.

15. A system for bandaging a patient (27) as set forth in Claim 14, wherein the tubular applicator handle (19, 21, 32, 33) has a flat section for attaching to the bandage (20, 31).

16. A system for bandaging a patient (16, 27) as set forth in any preceding claim, wherein the system includes a pair of applicator handles, (7, 8, 19, 21, 32, 33), the handles being joinable to opposite edge areas of the flexible bandage (1, 20, 31, 36).

17. A system for bandaging a patient as set forth in any preceding claim, wherein the applicator handle is preattached to the bandage (36), and both the applicator handle (37, 38) and the flexible bandage are sterile and inside of a sterility protecting package (35).

18. A system for bandaging a patient (16, 27) as set forth in any preceding claim, wherein the flexible bandage (1, 20, 31, 36) is rectangular.

19. A system for bandaging a patient (16, 27) as set forth in any preceding claim, wherein the bandage has a removable liner (5) protecting the adhesive prior to use.

20. A system for bandaging a patient (16, 27) comprising: a flexible bandage (1, 20, 31, 36) having a transparent backing (2, 25) that is permeable to water vapour, but impermeable to liquid water and bacteria; a transparent flexible adhesive (3, 26) continuously covering one surface of the backing so that the entire bandaging area of the flexible bandage can be directly adhered to a patient (16, 27); a removable liner (5, 24) protecting the adhesive of the flexible bandage prior to use; said flexible bandage being generally rectangular in shape and having graspable edge portions on at least two opposed edges of the bandage; and a separate applicator handle means (7, 8, 19, 21, 32, 33, 37, 38) substantially less flexible than the bandage which is joinable to the opposed graspable edge portions of the bandage for control of the bandage during application, said applicator handle means being removable from the bandage so as not to interfere with the flexible functioning of the bandage on a patient's anatomy.

**Patentansprüche**

1. System zum Verbinden eines Patienten (16, 27) umfassend: eine flexible Bandage bzw. einen Verband (1, 20, 31, 36) mit einer mit einem Klebstoff (3, 26) versehenen Unterlagschicht (2, 25); und einen getrennten Applikatorhandgriff (7, 8, 19, 21, 32, 33, 37, 38), der im wesentlichen weniger flexibel ist als der Verband und an diesen Verband für die Steuerung bzw. Führung des Verbandes während des Aufbringens anfügbar ist, wobei der Applikatorhandgriff aus dem Verband entfernbar ist, um nicht die flexible Funktion der Bandage bzw. des Verbandes auf der Anatomie eines Patienten zu behindern.

2. System zum Verbinden eines Patienten (16, 27) nach Anspruch 1, worin der flexible Verband (1, 20, 31, 36) ein Wundverband bzw. eine Auflage für eine Wunde ist.

3. System zum Verbinden eines Patienten (16, 27) nach Anspruch 2, worin der flexible Verband (1, 20, 31, 36) eine Brandwundenauflage ist.

4. System zum Verbinden eines Patienten (16, 27) nach Anspruch 1, worin der flexible Verband (1, 20, 31, 36) ein chirurgisches Tuch ist.

5. System zum Verbinden eines Patienten (16, 27) nach Anspruch 4, worin der flexible Verband (1, 20, 31, 36) ein chirurgisches Inzisionstuch ist, das an bzw. bei einer chirurgischen Inzision (17) durchtrennbar bzw. zerteilbar ist.

6. System zum Verbinden eines Patienten (16, 27) nach einem der vorhergehenden Ansprüche, worin der Verbund (1, 20, 31, 36) durchlässig für Wasserdampf, jedoch undurchlässig für flüssiges Wasser und Bakterien ist.

7. System zum Verbinden eines Patienten (16, 27) nach einem der vorhergehenden Ansprüche, worin die Unterlagschicht (2, 25) und der Klebstoff (3, 26) transparent sind, um einen Patienten (16) unter dem Verband betrachten zu können.

8. System zum Verbinden eines Patienten (16, 27) nach einem der vorhergehenden Ansprüche, worin der Klebstoff (3, 26) eine kontinuierliche Beschichtung auf der Unterlagschicht (2, 25) bildet, sodaß der Klebstoff dazu ausgebildet ist, die Unterlagschicht direkt auf einem Wund- oder Inzusionsbereich zu befestigen.

9. System zum Verbinden eines Patienten (16, 27) nach einem der vorhergehenden Ansprüche, worin der Handgriff (7, 8, 19, 21) starr ist.

10. System zum Verbinden eines Patienten (16) nach einem der vorhergehenden Ansprüche, worin der Handgriff (7, 8) ein Paar von bewegbaren Backen (9, 10) zum vorübergehenden Ergreifen des Verbandes (19) aufweist.

11. System zum Verbinden eines Patienten (16) nach Anspruch 10, worin eine Klinke (12) auf den Backen vorgesehen ist.

12. System zum Verbinden eines Patienten (27) nach einem der Ansprüche 1 bis 9, worin der Applikatorhandgriff (19, 21) am Verband (20) mittels eines Klebstoffs (23) befestigt ist.

13. System zum Verbinden eines Patienten nach einem der Ansprüche 1 bis 9 und 12, worin der Applikatorhandgriff (19, 21, 32, 33) biegbar ist.

14. System zum Verbinden eines Patienten (27) nach einem der Ansprüche 1 bis 9, 12 und 13, worin der Applikatorhandgriff (19, 21, 32, 33) röhrenförmig ist.

15. System zum Verbinden eines Patienten (27) nach Anspruch 14, worin der röhrenförmige Applikatorhandgriff (19, 21, 32, 33) einen flachen Abschnitt für die Befestigung am Verband (20, 31) aufweist.

16. System zum Verbinden eines Patienten (16, 27) nach einem der vorhergehenden Ansprüche, worin das System ein Paar von Applikatorhandgriffen (7, 8, 19, 21, 32, 33) umfaßt, welche Handgriffe an gegenüberliegende Randbereiche des flexiblen Verbandes (1, 20, 31, 36) anfügbar sind.

17. System zum Verbinden eines Patienten nach einem der vorhergehenden Ansprüche, worin der Applikatorhandgriff an dem Verband (36) vorherbefestigt wird und sowohl der Applikatorhandgriff (37, 38) und der flexible Verband steril sind und sich innerhalb einer sterilitätsgeschützten Packung (35) befinden.

18. System zum Verbinden eines Patienten (16, 27) nach einem der vorhergehenden Ansprüche, worin der flexible Verband (1, 20, 31, 36) rechteckig ist.

19. System zum Verbinden eines Patienten (16, 27) nach einem der vorhergehenden Ansprüche, worin der Verband eine entfernbare Deckschicht (5) zum Schutz des Klebstoffs vor der Verwendung aufweist.

20. System zum Verbinden eines Patienten (16, 27) umfassend: einen flexiblen Verband (1, 20, 31, 36) mit einer transparenten Unterlagschicht (2, 25), die für Wasserdampf durchlässig, jedoch für flüssiges Wasser und Bakterien undurchlässig ist; einen transparenten flexiblen Klebstoff (3, 26), der kontinuierlich eine Oberfläche der Unterlagschicht bedeckt, sodaß der gesamte Verbandbereich des flexiblen Verbandes direkt auf einen Patienten (16, 27) geklebt werden kann; eine entfernbare Deckschicht (5, 24) zum Schutz des Klebstoffs auf dem flexiblen Verband vor der Verwendung; wobei der genannte flexible Verband im allgemeinen von rechteckiger Form ist und ergreifbare Randabschnitte auf wenigstens zwei gegenüberliegenden Rändern des Verbandes aufweist; und eine getrennte Applikatorhandgriff-Einrichtung (7, 8, 19, 21, 32, 33, 37, 38), die im wesentlichen weniger flexibel ist als der Verband und an die gegenüberliegenden ergreifbaren Randabschnitte des Verbandes für die Steuerung bzw. Führung des Verbandes während der Applikation anfügbar ist, wobei die genannte Applikatorhandgriff-Einrichtung aus dem Verband entfernbar ist, um nicht die flexible Funktion der Bandage bzw. des Verbandes auf der Anatomie eines Patienten zu behindern.

**Revendications**

1. Système de bandage d'un patient (16, 27) comprenant un bandage flexible (1, 20, 31, 36) ayant un support (2, 25) muni d'un adhésif (3, 26), et un manche applicateur séparé (7, 8, 19, 21, 32, 33, 37, 38) qui est substantiellement moins flexible que le bandage et il est assemblé à un tel bandage pour contrôler le bandage lors de sa mise en place, ledit manche applicateur pouvant être retiré du bandage de sorte à ne pas interférer avec la flexibilité de bandage sur l'anatomie du patient.

2. Système de bandage d'un patient (16, 27) comme défini à la revendication 1, caractérisé par le fait que le bandage flexible (1, 20, 31, 36) est un pansement pour une plaie.

3. Système de bandage d'un patient (16, 27) comme défini à la revendication 2, caractérisé par le fait que le bandage flexible (1, 20, 31, 36) est un pansement pour une brûlure.

4. Système de bandage d'un patient (16, 27) comme défini à la revendication 1, caractérisé par le fait que le bandage flexible 1, 20, 31, 36) est un drap chirurgical.

5. Système de bandage d'un patient (16, 27) comme défini à la revendication 4, caractérisé par le fait que le bandage flexible (1, 20, 31, 36) est un drap chirurgical à inciser qui peut être coupé par une incision chirurgicale (17).

6. Système de bandage d'un patient (16, 27) comme défini par l'une quelconque des revendications précédentes, caractérisé par le fait que le bandage (1, 20, 31, 36) est perméable à la vapeur d'eau, mais imperméable à l'eau liquide et aux bactéries.

7. Système de bandage d'un patient (16, 27) comme défini par l'une quelconque des revendications précédentes, caractérisé par le fait que le support (2, 25) et l'adhésif (3, 26) sont transparents pour pouvoir voir un patient (16) au-dessus du bandage.

8. Système de bandage d'un patient (16, 27) comme défini par l'une quelconque des revendications précédentes, caractérisé par le fait que l'adhésif (3, 26) forme une couche continue sur le support (2, 25) de sorte que l'adhésif est adapté pour assurer le support directement sur une plaie ou une région d'incision.

9. Système de bandage d'un patient (16, 27) comme défini par l'une quelconque des revendications précédentes, caractérisé par le fait que le manche (7, 8, 19, 21) est rigide.

10. Système de bandage d'un patient (16) comme défini par l'une quelconque des revendications précédentes, caractérisé par le fait que le manche (7, 8) a une paire de mâchoires mobiles (9, 10) pour saisir temporairement le bandage (1).

11. Système de bandage d'un patient (16) comme défini à la revendication 10, caractérisé par le fait que les mâchoires sont munies de moyens de verrouillage (12).

12. Système de bandage d'un patient (27) comme défini par l'une quelconque des revendications 1 à 9, caractérisé par le fait que le manche applicateur (19, 21) est assuré sur le bandage (20) par un adhésif (23).

13. Système de bandage d'un patient comme défini par l'une quelconque des revendications 1 à 9 et 12, caractérisé par le fait que le manche applicateur (32, 33) est pliable.

14. Système de bandage d'un patient (27) comme défini par l'une quelconque des revendications 1 à 9, 12 et 13, caractérisé par le fait que le manche applicateur (19, 21, 32, 33) est tubulaire.

15. Système de bandage d'un patient (27) comme défini par la revendication 14, caractérisé par le fait que le manche applicateur tubulaire (19, 21, 32, 33) a une section plate pour être attaché au bandage (20, 31).

16. Système de bandage d'un patient (16, 27) comme défini par l'une quelconque des revendications précédentes, caractérisé par le fait que le système comprend une paire de manches applicateurs (7, 8, 19, 21, 32, 33), les manches étant assemblables aux régions d'extrémité opposées du bandage flexible (1, 20, 31, 36).

17. Système de bandage d'un patient comme défini par l'une quelconque des revendications précédentes, caractérisé par le fait que le manche applicateur est attaché préalablement au bandage (36), et qu'aussi bien le manche applicateur (37, 38) et le bandage flexible sont stérilisés et à l'intérieur d'un emballage de protection stérile (35).

18. Système de bandage d'un patient (16, 27) comme défini par l'une quelconque des revendications précédentes, caractérisé par le fait que le bandage flexible (1, 20, 31, 36) est rectangulaire.

19. Système de bandage d'un patient (16, 27) comme défini par l'une quelconque des revendications précédentes, caractérisé par le fait que le bandage à une feuille amovible (5) de protection protégeant l'adhésif avant l'utilisation.

20. Système de bandage d'un patient (16, 27) comprenant: un bandage flexible (1, 20, 31, 36) ayant un support transparent (2, 25) qui est perméable à la vapeur d'eau, mais imperméable à l'eau liquide et aux bactéries; un adhésif flexible transparent (3, 26) couvrant de manière continue une surface du support, de sorte que toute la surface de bandage du bandage flexible peut adhérer directement sur un patient (16, 27); une feuille de protection amovible (5, 24) protégeant l'adhésif du bandage flexible avant l'utilisation; ledit bandage flexible étant généralement de forme rectangulaire et ayant deux parties d'extrémité saisissables sur au moins deux extrémités opposées du bandage; et un moyen de tenue applicateur séparé (7, 8, 19, 21, 32, 33, 37, 38) substantiellement moins flexible que le bandage qui peut être assemblé aux parties d'extrémité saisissables opposées du bandage pour le contrôle du bandage lors de son application, ledit moyen de tenue applicateur pouvant être retiré du bandage pour ne pas interférer avec la flexibilité du bandage sur l'anatomie du patient.

EP 0 120 840 B1

Fig.1

Fig.2

Fig.3

Fig.4

Fig.5

Fig.6

Fig.7

2/2

*Fig. 8*

9

*19*

*20*

9

*21*

*Fig. 9*

*19*

*23*

*24*

*Fig. 10*

*21*

*19*

*25*

*29*

*27*

*26*

*28*

*31*

*32*

*Fig. 11*

*33*

*Fig. 13*

*35*

*37*

*36*

*38*

*Fig. 12*

*33*

*31*

*Fig. 14*

*39*

*40*